# EUROPÄISCHE PATENTANMELDUNG

(11) **EP 2 163 239 A1**
(43) Veröffentlichungstag der Anmeldung: **17.03.2010**
(21) Anmeldenummer: 08156939.4
(22) Anmeldetag: 27.05.2008
(51) Int. Cl.: A61K 9/20, A61K 35/00, A61K 48/00

(54) **Produkte, die Biopartikel enthalten, Verfahren zu ihrer Herstellung**

(71) Anmelder: Qiagen GmbH, 40724 Hilden (DE)
(72) Erfinder: Himmelreich, Ralf, 40764, Langenfeld (DE)
(74) Vertreter: Gille Hrabal Struck Neidlein Prop Roos

(57) **Zusammenfassung**

Offenbart wird ein im wesentlichen festes Produkt, enthaltend mindestens eine Art Biopartikel sowie übliche pharmazeutische Hilfsstoffe, ein Verfahren zu seiner Herstellung und seine Verwendung als interner Standard in Nukleinsäurepräparations- und Nukleinsäurenachweis-Verfahren.

## Beschreibung

Die vorliegende Erfindung betrifft im wesentlichen feste Produkte, die mindestens eine Art Biopartikel sowie übliche pharmazeutische Hilfsstoffe enthalten, ein Verfahren zu ihrer Herstellung und ihre Verwendung als interner Standard in Nukleinsäurepräparations- und Nukleinsäurenachweis-Verfahren, insbesondere in mikrofluidischen Vorrichtungen.

### Hintergrund der Erfindung

Es ist zunehmend von großer Bedeutung, Biopartikel wie Bakterien, Viren, Pilze, Protozoen oder ähnliche in Proben unterschiedlicher Herkunft nachzuweisen. So ist es beispielweise für die Auswahl eines geeigneten Behandlungsverfahrens oder Therapeutikums für an Infektionen erkrankte Patienten sehr bedeutsam, zu bestimmen, welcher Krankheitserreger vorliegt. Bisher wurden z.B. bei Verdacht auf bakterielle Infektionen zu diesem Zweck häufig Proben wie .B. Blut-oder Urinproben inkubiert und nach einigen Tagen anhand langwieriger Tests mit verschiedenen Antibiotika bestimmt, welche Erreger vorliegen und welche Therapeutika ggf. geeignet zur Behandlung wären. Häufig wird dadurch wertvolle Zeit verloren. Auch ist es zunehmend wichtig, Biopartikel in Proben wie Lebensmitteln, Trinkwasser o.ä. zur Qualitätskontrolle nachzuweisen. Auch die Anschläge mit dem Anthrax (Milzbrand)-Erreger *Bacillus anthracis* in den USA haben verdeutlicht, dass sehr schnelle und zuverlässige Analyseverfahren für Mikroorganismen dringend notwendig sind.

Es bestehen daher schon seit längerer Zeit Bestrebungen, schnellere Diagnoseverfahren zu entwickeln, die schon nach einigen Minuten oder Stunden die Bestimmung von Erregern ermöglichen.

Eine Möglichkeit dafür besteht darin, die Nukleinsäuren der in einer Probe enthaltenen Erreger freizusetzen, diese anschließend spezifisch zu amplifizieren und dann nachzuweisen. Hierzu sind folgende Schritte erforderlich:
- Materialentnahme (z.B. Blut, Sputum, Urin, Faeces, Liquor, sowie Abstrichproben aus Mundhöhle, Nasenraum, Genitaltrakt)
- Lyse(Aufschließen der Zellen)
- Nukleinsäure-Präparation
- Nukleinsäure-Amplifikation
- Detektion der amplifizierten Nukleinsäuren

Unter Aufschließen/Lyse von Zellen wird das Aufbrechen von Zellen verstanden, wobei Nukleinsäuren aus Zellenfrelgesetzt werden. Des weiteren werden Proteine aus dem Zellinneren freigesetzt.

Zellen sind unterschiedlich schwierig aufzuschließen. Kultivierte Zellkulturen können in der Regel einfach mit vergleichsweise die Erbinformation schonenden Mitteln lysiert werden. Sporen sind dagegen vergleichsweise schwierig aufzuschließen. Die für die Lyse von solchen Zellen benötigten Mittel brechen nicht nur die Zellen auf, sondern fragmentieren auch Erbinformationen in großem Ausmaß.

Ein Beispiel für einfach aufzuschließende Zellen sind weiße Blutkörperchen. Diese können vergleichsweise schonend lysiert werden, beispielsweise durch das Enzym Proteinase K in Anwesenheit eines Detergenz (z.B Natrium Dodecylsulfat oder Triton X-100).

Bei jedem Zellaufschluss werden Nukleinsäure geschert und durch Doppelstrangbrüche im Erbmaterial fragmentiert. Allerdings erfolgt die Fragmentierung der Erbinformation nicht derart umfangreich, dass dadurch die gewünschten Analysen gefährdet wären.

Je größer das Ausmaß an Fragmentierung der Nukleinsäuren ist, umso problematischer ist es, die gewünschten Analysen durchzuführen. Es ist also ein Ziel bei dem Aufschluss von Zellen, Doppelstrangbrüche auf ein verträgliches Maß zu reduzieren.

Sollen schwer aufzuschließende Zellen lysiert werden, so geschieht dies beispielsweise durch Zufuhr von Hitze. Dann werden schwer aufzuschließende Zellen beispielsweise 10 Minuten lang bei 95°C behandelt. Typischerweise werden viele Bakterien mit Hilfe von Hitze lysiert.

Ein weiteres Verfahren ist die Lyse mittels Ultraschall, welches im Fall von schwierig aufzuschließenden Zellen angewendet wird. Ultraschall fragmentiert Erbinformationen allerdings besonders umfangreich. Daher wird Ultraschall üblicherweise nur dann eingesetzt, wenn andere Verfahren versagen. Mit Hilfe von Ultraschall werden beispielsweise Sporen lysiert, die besonders widerstandsfähig sind.

Ein drittes Verfahren für die Lyse von schwierig aufzuschließenden Zellen stellt das Mahlen mittels Glaskugeln dar. Mit Hilfe von Glaskugeln werden Bakterien und Pilze aufgeschlossen. Der Aufschluss mittels Glaskugeln (in Fachkreisen auch als "Bead Beating" bekannt) verläuft vergleichsweise schonend im Vergleich zu Ultraschall und Hitze. In sämtlichen drei Fällen werden jedoch die Erbinformationen unterschiedlich stark fragmentiert.

Soll mit Glaskugeln aufgeschlossen werden, so wird in der Regel eine Rüttel- oder Schüttelapparatur so zum Beispiel ein sogenannter Vortex^{®}-Rüttler verwendet. Ein oder mehrere aus Kunststoff bestehende Röhrchen werden mit einer wässrigen Lösung, die einen Lysepuffer umfasst, dem Zellmaterial und Glaskügelchen gefüllt, verschlossen und in die Rüttel- oder Schüttelapparatur gestellt und mittels der Apparatur beispielsweise für fünf Minuten geschüttelt. Ein solches Verfahren wird auch als "Vortexen mit Glasbeads" oder als "Bead Beating" bezeichnet.

Ist der Zellaufschluss abgeschlossen, so werden das oder die Röhrchen entnommen, die Röhrchen bei Bedarf zentrifugiert, um Glaspartikel von der Flüssigkeit zu trennen, und der flüssige Inhalt wird in ein weiteres Röhrchen gegeben. Hier wird das lysierte Zellmaterial mit weiteren Pufferlösungen behandelt, um die Nukleinsäure zu isolieren.

Eine Lyse mittels Glaskügelchen wird beispielsweise in der Druckschrift DE 10 2004 032 888 B4 beschrieben.

Nach der Lyse können die freigesetzten Nukleinsäuren bevorzugt aus dem erhaltenen Gemisch isoliert werden, z.B. durch Festphasenextraktion an geeigneten Trägern wie z.B. Silika, Glasfasermembranen oder durch Absorption an Magnetpartikeloberflächen. Anschließend wird mit geeigneten Lösungsmitteln, insbesondere mit Waschpufferlösungen, gewaschen, und dann werden die Nukleinsäuren mit geeigneten Elutionspuffer eluiert. Diese Techniken sind Fachleuten gut bekannt, und geeignete Adsorptionsmaterialien, Waschpuffer und Elutionspuffer können leicht durch Testen ausgewählt werden.

Die Amplifikation von Nukleinsäuren wird üblicherweise durch PCR (Polymerase Chain Reaction) durchgeführt. Andere Amplifizierungsverfahren sind z.B. Ligase Chain Reaction (LCR), gap-filling LCR (Gap-LCR), Nukleinsäuresequenz-basierte Amplifizierung (NASBA) und Transkriptionsvermittelte Amplifizierung (TMA). Diese Verfahren sind im Stand der Technik gut bekannt.

Die PCR ist eine enzymatische Methode zur Synthese spezifischer Nukleinsäuresequenzen unter Verwendung von zwei Oligonukleotid-Primern (Sonden), die mit entgegengesetzten Strängen hybridisieren und einen interessierenden Abschnitt einer Target-Nukleinsäure flankieren. Eine Serie von wiederholten Reaktionsschritten, welche Templat-Denaturierung, Primer-Annealing und Verlängerung der hybridisierten Primer durch die DNA-Polymerase umfassen, resultiert in exponentieller Akkumulation des spezifischen Target-Fragments, dessen Enden durch die 5'-Enden der Primer definiert sind. Das PCR-Verfahren verwendet wiederholte Zyklen der DNA-Synthese zur Replikation der Target-Nukleinsäure. In der grundlegenden Ausführungsform umfasst die PCR folgende Schritte:
- Zugabe spezifischer Primer zu einer Probe, von der vermutet wird, dass sie die Target-Nukleinsäure enthält. Die Primer sind so gewählt, dass sie an komplementäre DNA-Stränge binden, die in entgegengesetzten Strängen der DNA vorliegen. Die Target-Nukleinsäure ist innerhalb der Primerbindungsstellen lokalisiert und ist ein Marker des nachzuweisenden Mikroorganismus.
- Zugabe der vier Desoxynukleosid-Triphosphate, Magnesiumsulfathaltiger Puffer, Polymerase-Enzyme und von verschiedenen Additiven und Cosolventien, Mischen mit der Probe und den Primern
- Denaturieren der Probe durch Erwärmen, Trennung der DNA in zwei komplementäre Stränge
- Abkühlung, dadurch Bindung (Hybridisierung) der Primer an die jeweiligen Bindungsstellen an den komplementären DNA-Strängen
- Primer-Extension auf dem DNA-Templat durch DNA-Polymerase.

Diese drei Schritte Denaturierung, Hybridisierung und Extension werden 40 bis 50 Mal wiederholt und resultieren in einer exponentiellen Amplifikation der Targetsequenzen.

Zum Nachweis der Amplifizierungsprodukte werden geeignete Sonden zugesetzt, die z.B. mit Markern wie Fluoreszenzmarkern (Fluorophoren) markiert sind. Es handelt sich um Oligonukleotide, die komplementär zur Target-DNA sind. Diese Sonden hybridisieren mit der Target-DNA und ermöglichen dann später deren Nachweis, z.B. durch Fluoreszenzspektroskopie.

Die PCR-Technik ist z.B. umfassend in PCR Technology: Principles and Applications for DNA Amplification Erlich, Hrsg. (1992); PCR Protocols: A Guide to Methods and Applications, Innis et al., Hersg. (1990), R.K. Saiki et al., Science 230 :1350 (1985) und US 4,683,202 beschrieben, deren Offenbarung vollinhaltlich hier aufgenommen wird.

Die klassische PCR-Reaktion ist eine Endpunkt-PCR. Dadurch ist es möglich, die Anwesenheit oder Abwesenheit einer Target-DNA nachzuweisen, die Methode gibt aber keine Auskunft über die Anfangskonzentration der Target-DNA.

In neuerer Zeit wurde die Real-Time PCR (RT-PCR; Echtzeit-Polymerase-Chain-Reaktion; auch quantitative PCR (qPCR) genannt) entwickelt. Diese ermöglicht das gleichzeitige Verfolgen von mehreren Nukleinsäure-Amplifikationsreaktionen durch PCR, wobei die akkumulierten Daten zur quantitativen Bestimmung der Ausgangskonzentration einer Target-Nukleinsäuresequenz verwendet werden. Daher wird diese PCR-Raktion auch als Multiplex-PCR bezeichnet. Offenbart ist die Real-Time PCR z.B. in EP-A-0 512 34, EP-A-0 640 828, EP-A-0 519 338 (F. Hoffmann- La Roche AG). Es werden hierfür kommerziell Systeme angeboten, z.B. TaqMan® (Roche Molecular Systems, Inc., Branchburg Township, New Jersey). Das Verfahren ermöglicht die schnelle und präzise Quantifizierung der Ausgangskopienzahl über einen sehr weiten Konzentrationsbereich. Es ist geeignet für viele Anwendungen wie Genexpressionsstudien, Sequenz- und Mutationsanalysen, Überprüfung von Virustitern, den Nachweis pathogener oder genetisch modifizierter Organismen. Ein Kit für Real-Time PCR mit sequenzspezifischen Sonden ist z.B. erhältlich von Eppendorf AG, Hamburg (RealMasterMix Probe^{®}) oder von artus GmbH, Hamburg (RealArt^{®} PCR Kits).

Bei der TaqMan® PCR sind die Oligonukleotidsonden so ausgelegt, dass sie an der Target-Nucleinsäuresequenz stromaufwärts vom Extensionprimer binden. Jede Oligonukleotidsonde ist am 5'-Ende mit einem Reportermolekül wie einem Fluorophor und am 3'-Ende mit einem Reportermolekül-Quencher markiert. Die markierten Sonden werden gemeinsam mit den Primern und der Probe zur PCR-Reaktionsmischung gegeben. Nach der Denaturierung wird die Reaktionsmischung abgekühlt, wobei die markierten Sonden bevorzugt an die Primer binden. Als nächstes wird bis auf die optimale Temperatur zur Primerbindung und Extension abgekühlt. Im Verlauf der Polymerisation, wenn die DNA-Polymerase entlang dem Target-Nukleinsäurestrang vom 3'-Ende zum 5'-Ende fortschreitet, wobei Nukleotide an den wachsenden Primer angefügt werden, trifft der Primer auf die 5'-Enden der zuvor an den Target-Nukleinsäurestrang gebundenen markierten Sonden. Wenn die DNA-Polymerase auf diese markierten Sonden trifft, übt sie ihre 5'-3'-Endonucleaseaktivität aus und baut die markierten Sonden ab. Fluorophore und Quencher werden dabei n die Reaktionsmischung freigesetzt. Der TaqMan® Assay ist so ausgelegt, dass er Reportermoleküle nicht nachweist, die innerhalb einer vorherbestimmten Nähe des Quenchermoleküls vorliegen. Daher wird in der PCR-Mischung zu Beginn kein Fluoreszenz aufgrund des Reportermoleküls nachgewiesen, weil die Fluoreszenz des Reportermoleküls vom in räumlicher Nähe befindlichen Quenchermolekül gequencht wird. Während der PCR werden die 5'-fluoreszenzmarkierten Sonden freigesetzt und dabei von den 3'-Fluoreszenzquenchern getrennt. Nach der Freisetzung wird der Fluoreszenzmarker nicht mehr gequencht und kann daher durch Fluoreszenzspektrometrie oder durch andere geeignete Mittel nachgewiesen werden. In der Reaktionsmischung vorhandene ungebundene Sonden stören nicht, da sie gequencht bleiben. Unspezifisch an Nukleinsäuresequenzen, die nichts mit der Target-Nukleinsäure zu tun haben, gebundene markierte Sonden stören ebenfalls nicht, da sie gebunden und daher gequencht bleiben. Daher ist jedes freie Reportermolekül, das in der Reaktionsmischung nachgewiesen wird, direkt proportional zu der Menge der ursprünglich spezifisch gebundenen markierten Sonde, und daher der Target-Nukleinsäure. Zum Nachweis unterschiedlicher Target-Nukleinsäuren in einer Probe werden Sonden mit unterschiedlichen Fluoreszenz-Reportermolekülen verwendet, die in unterschiedlichen Wellenlängenbereichen fluoreszieren und daher den gleichzeitigen Nachweis verschiedener Target-DNAs ermöglichen. Als Fluoreszenz-Reporter sind z.B. 6-FAM, VIC, Bodipy-TMR, JOE und HEX bekannt und geeignet, als Quencher TAMRA, MBG, Black Hole Quencher 1 (BHQ1), Black Hole Quencher 2 (BHQ2), Black Hole Quencher 3 (BHQ3), BodyPi 564/570 und Cy5 oder ähnliche. Der Fachmann wählt die Reporter und Quencher geeignet aus, bevorzugt so, dass eine gegenseitige Überlagerung der Fluoreszenzemissionen ("Bleed through") vermieden wird. Z.B. ist die Kombination 6-FAM/BHQ1 besonders geeignet.

Neben Fluoreszenzmarkern können auch radioaktive Marker, chemilumineszente Marker, paramagnetische Marker, Enzyme und Enzymsubstrate als Marker verwendet werden.

Im Anschluss an die Amplifikation erfolgt die Detektion der Nukleinsäuren durch Fluoreszenzspektrometrie, wie vorstehend beschrieben, oder bei Verwendung anderer Marker durch entsprechende andere Detektionsverfahren.

Die genannten Nukleinsäurepräparations- und -amplifikations- sowie' detektionsverfahren können im Labormaßstab, d.h. im Mikro- oder Millilitermaßstab durchgeführt werden. Alternativ wurden in jüngerer Zeit mikrofluidische Verfahren entwickelt.

Unter Mikrofluidik wird die Handhabung und das Arbeiten mit kleinen Flüssigkeitsmengen verstanden, die ein um Zehnerpotenzen geringeres Volumen aufweisen als normale Flüssigkeitstropfen.

Vorrichtungen zur Durchführung mikrofluidischer Verfahren, die eine Probe bis zum gewünschten Messergebnis verarbeiten, werden auch als Lab-on-a-chip (LoC) bezeichnet, also Einweg-Verbrauchsmaterial als Labor auf einem Chip in der Größe etwa einer Kreditkarte, mit dem molekularbiologische Bestimmungen vorgenommen werden, z.B. die Bestimmung von Erregern einer Infektion, Nahrungsmittelkontrolle, Veterinärdiagnostik, Analyse biologischer Kampfstoffe, Umweltanalytik. Für die Analyse ist nur geringer Personalaufwand und keine spezielle Ausbildung oder Einarbeitung zur Bedienung erforderlich. LoC werden in ein Betreiberinstrument von der Größe eines Videorecorders eingelegt, das dann das Ergebnis ausgibt. Ein LoC vereinigt mikrofluidische Funktionen zur Probenextraktion und Anreicherung des Analyten, zur Signalverstärkung (Amplifikation) und Detektion in einem Chip. Die Bedienung des LoC und der Bedieneinheit ist sehr einfach, und die Analyse erfolgt extrem schnell verglichen mit konventionellen Analyseverfahren (30-60 Minuten Gesamtprozesszeit gegenüber mindestens 6 Stunden).

Ein großes Problem bei den Nukleinsäurepräparations- und -amplifikationsschritten sowohl im Labormaßstabe als auch mit mikrofluidischen Vorrichtungen ist es, eine zuverlässige Kontrolle des Verfahrens einzubauen, um insbesondere falsch-negative Ergebnisse zu vermeiden. Falsch-negativ bedeutet, dass die Target-Nukleinsäure zwar in der Probe vorliegt, weil z,B. ein bestimmter Erreger vorhanden ist, aber durch Fehler im Test nicht nachgewiesen wird. Dabei können sowohl die Lyse der Zellen, die Isolierung der Nukleinsäuren als auch die Amplifikation fehleranfällig sein. Insbesondere die PCR ist empfindlich gegen nachteilige Wirkungen von Inhibitoren, von denen viele häufig in biologischen Proben vorliegen, wie z.B. Häm und seine metabolischen Produkte, saure Polysaccharide, Detergentien und chaotrope Salze. Aber auch die Lyse der Zellen verläuft nicht immer zuverlässig, besonders bei schwer zu lysierenden Zellen, wie vorstehend beschrieben.

Es wurde daher bereits vorgeschlagen (WO 02/052041, WO 2005/04762), der PCR interne Kontrollsubstanzen zuzugeben, die so angelegt sind, dass die korrekte Durchführung der Amplifikation bestätigt wird. Der interne Standard kann der Probe vor dem ersten Bearbeitungsschritt zugesetzt werden, z.B. bereits vor der Reinigung oder der Lyse, oder auch vor der PCR. l.a. wird ein synthetisches Oligonukleotid-Konstrukt hergestellt, dass von der Target-Region des Testdetektionssystems verschieden ist (internes Kontroll-Target). Die geeignete Auswahl der internen Kontrolle ist sehr komplex und schwierig.

Im Stand der Technik als interne Kontrollstandards bekannt sind gereinigte Nukleinsäuren, Protein-komplexierte Nucleinsäuren ("armored RNA" der Fa. Ambion), z.B. mit Capsid, oder inaktive Virusstandards.

Problematisch bei den gereinigten Nukleinsäuren ist ihre geringe Stabilität. Insbesondere RNA wird in Blut oder anderen biologischen Probematerialien innerhalb kürzester Zeit abgebaut. Zudem ermöglicht die Verwendung von Nukleinsäuren keine Kontrolle, ob die Lyse, also der Probenaufschluss inklusive Nukleinsäurefreisetzung korrekt verlaufen ist, sondern nur die Kontrolle der Amplifikation.

Die "armored RNA" ist zwar deutlich stabiler als "nackte" Nukleinsäuren, ist aber deutlich leichter zu lysieren als intakte Zellen und daher nicht mit diesen vergleichbar und als Kontrolle der Lyse nicht gut geeignet.

Die inaktiven Virusstandards sind im Grunde Vaccine und enthalten Chemikalien, insbesondere Formaldehyd, die die Oberflächenstruktur der Viren durch chemische Kreuzvernetzung gegenüber aktiven Formen verändern. Daher ist das Lyseverhalten nicht vergleichbar mit dem von lebenden Viren, und die inaktiven Virusstandards sind als Kontrolle für die Lyse nicht unbedingt geeignet.

WO 03/02959 betrifft durch Gefriertrocknen erhaltene Produkte, die bestimmte definierte Mengen Biopartikel enthalten. Die Anmeldung deckt nach Angabe des Anmelders in der letzten Eingabe im EP-Verfahren vom 22.8,2006 das Produkt BioBall® ab, das gefriergetrocknete Kügelchen darstellt, die für die quantitative mikrobiologische Qualitätskontrolle eingesetzt werden. Sie werden bisher in der Lebensmittel-, Pharma-, Wasser-und Abwasserindustrie eingesetzt, können aber auch für Kosmetik, "personal care", klinische Labors and Forschungs- und akademische Institutionen verwendet werden. Weitere Bestandteile der Kügelchen oder Anwendung als interne Kontrollen bei Nukleinsäurepräparationen ist nicht offenbart. BioBalls® sind mit verschiedenen Bakterien verfügbar, z.B. Enterokokken , *E*. *coli*, Salmonellen. Es wird u.a. ein Set von im wesentlichen festen Produkten offenbart, die eine definierte Anzahl von 1 bis 1000 Mikroorganismen enthalten, ausgewählt aus Viren, Bakterien, Hefen, Pilzen, Parasiten, Protozoen, Zellen und Mischungen daraus, wobei die festen Produkte ohne Verlust an Mikroorganismen zwischen Behältern transferiert werden können, und die Mikroorganismen in einer Flüssigkeit freigesetzt werden können, und wobei die Abweichung der Zahl der Mikroorganismen zur definierten Anzahl höchstens 10 % beträgt. Der Sinn dieser Produkte liegt darin, eine genau definierte sehr geringe Anzahl von Mikroorganismen für Vergleichsproben in der Qualitätskontrolle bereitzustellen.

WO 2004/055205 offenbart die Verwendung von Zellen, viralen Partikeln, Organellen, Organellen, virale Partikel, Parasiten oder bakterielle Zellen umfassenden Zellen, die mindestens eine Nukleinsäuresequenz umfassen, die als internes Kontroll-Target für Nukleinsäureassays dient, als interne Kontrolle. PCR ist als bevorzugt offenbart. Als Zellen sind Bakterien, Viren, Pilze, Parasiten, eukaryontische Zellen oder Pflanzenzellen oder auch Sporen genannt. Es wird als besonders vorteilhaft beschrieben, dass sowohl die Nukleinsäurepräparation als auch die Amplifikation durch direkte Zugabe der Zellen zu der Testprobe überprüft werden kann. Es können genetisch veränderte Zellen (d.h. solche, in die die Kontroll-DNA-Sequenz durch gentechnische Methoden eingebaut wurde) oder natürliche Zellen wie z.B. Sporen von *B. globigii* als Kontrollzellen eingesetzt werden. Feste Präparate sind nicht offenbart, sondern Suspensionen, die die durch Einschleusen der Kontroll-DNA genetisch veränderten Sporen enthalten.

WO 02/18635 offenbart die Verwendung von nicht lebensfähigen Partikeln, die eine interne Nukleinsäuresequenz umfassen, als interne Kontrolle für nukleinsäurebasierte Analysen. Die Partikel sind insbesondere Liposomen.

DE 21 40 747 offenbart ein Verfahren zum Mischen von festen biologischen Stoffen mit anderen festen Stoffen durch Gefriertrocknen in Gegenwart eines Zuckers.

WO 99/06594 offenbart ein Verfahren zur Herstellung stabil eingekapselter Nukleinsäuren, die zur Überwachung aller Schritte von Nukleinsäurepräparationen verwendbar sind. Die beschriebenen Zellen werden nach Einschleusen der Nukleinsäuren abgetötet, z.B. durch Stehenlassen bei Raumtemperatur oder mit chemischen Reagenzien.

EP 1 1 79 585 und WO 99/33559 offenbaren eine integrierte Kartusche zur Analyse von klinischen oder Umwelt-Flüssigkeiten, insbesondere zum Nachweis von DNA. Das System ist kommerziell von der Fa. Cepheid unter der Bezeichnung GeneXpert® erhältlich. Es gibt für verschiedene Assays unterschiedliche interne Kontrollen, z.B. für den Assay von MRSA (Methicillinresistenter *Staphylococcus aureus*) Sporen von *Bacillus globigii*; oder für virale RNA "armored RNA" der Fa. Ambion.

Die genannten internen Standards sind alle noch nicht zufriedenstellend. Zum einen können die meisten von ihnen nur für die Kontrolle der PCR, nicht aber für die der Lyse eingesetzt werden, da es sich nicht um intakte Mikroorganismen mit vergleichbarer Struktur wie die nachzuweisenden handelt. Zum anderen handelt es sich z.T. um instabile, nicht bei Normalbedingungen lagerfähige Produkte. Für eine einfache Verfahrensführung sind die z.T. bekannten Suspension oder Lösungen zudem nachteilig, weil sie aufwendiger und ungenauer zu dosieren und zuzugeben sind als z.B. feste Präparate.

Sowohl für Verfahren im Labormaßstabe als auch für mikrofluidische Verfahren besteht ein dringender Bedarf an zuverlässigen internen Standards, die die Kontrolle der korrekten Durchführung sowohl der Lyse als auch der Amplifikation ermöglichen. Für Labormaßstabverfahren ist dabei zu berücksichtigen, dass der interne Standard prinzipiell vor oder nach jedem Einzelschritt des Nachweisverfahrens zugegeben werden kann, oder dass auch verschiedenen Standards für jeden Schritt eingesetzt werden können. Bei mikrofluidischen Verfahren dagegen muss der interne Standard zu Beginn des Verfahrens zugegeben werden und für die Kontrolle aller Schritte geeignet sein, da eine Zugabe aufgrund der Verfahrensdurchführung in einem geschlossenen System in späteren Schritten nicht mehr möglich ist.

Wie vorstehend ausgeführt, gibt es bisher noch keine zufriedenstellenden internen Standards, die es ermöglichen, sämtliche Schritte eines Nachweisverfahrens für Biopartikel durch Analyse von dessen Nukleinsäuren (d.h. Präparation/Lyse der Zellen, Extraktion, Amplifikation und Detektion) zuverlässig zu kontrollieren.

Die Aufgabe der Erfindung besteht daher darin, einen geeigneten internen Standard für Nukleinsäurepräparations- und Nukleinsöurenachweis-Verfahren bereitzustellen.

### Offenbarung der Erfindung

Die Aufgabe wird gelöst durch das im wesentlichen festes Produkt gemäß Anspruch 1 , enthaltend mindestens eine Art Biopartikel sowie übliche pharmazeutische Hilfsstoffe. Bevorzugte Ausführungsformen sind in den Ansprüchen 2 bis 6 definiert. Ebenfalls beansprucht wird ein Herstellungsverfahren für das erfindungsgemäße Produkt sowie dessen Verwendung als interner Standard in Nukleinsäurepräparations- und Nukleinsäurenachweis-Verfahren.

Überraschenderweise ist es erfindungsgemäß erstmals gelungen, ein festes bei Normalbedingungen und Umgebungstemperatur gut lagerfähiges und stabiles Produkt zu erhalten, das Biopartikel umfasst und als interner Standard für Nukleinsäurepräparations- und Nukleinsäurenachweis-Verfahren verwendet werden kann. Es ist auf sehr einfache Art und Weise erhältlich, wobei keine aufwendigen und teuren Verfahrensschritte wie Gefriertrocknung notwendig sind.

### Beschreibung der Figuren

Figur 1 ist eine Photographie eines Ethidium-Bromid gefärbten Agarosegels, auf dem die Probeneluate aus Beispiel 2 elektrophoretisch aufgetrennt wurden. Bild A zeigt die Nukleinsäurepräparation für Blut bzw. PBS mit der Tablette A, Bild B die Nukleinsäurepräparation für Blut bzw. PBS mit Tablette B.

Figur 2 ist eine in Beispiel 3 erhaltene Graphik, die das Ergebnis der Real Time PCR-Quantifizierung der genomischen DNA von *Corynebacterium glutamicum* zeigt. Das Histogramm zeigt die durch PCR nachgewiesene Menge (pg) pro Reaktion bei einer Stichprobenmenge von N = 4.

Figur 3 ist eine in Beispiel 4 erhaltene Graphik, die die Treshold-Cycles (Ct) der quantitativen PCR einzeln präparierter Blut-Proben zeigt. Für jede Probe wurde eine *Corynebacterium glutamicum*-spezifische und eine Human-DNAspezifische (β-Aktin-Gen) PCR durchgeführt.

Figur 4 ist eine Graphik, die das Ergebnis der quantitativen PCR zum Nachweis von *B*. *subtilis*-DNA aus Beispiel 5 mit unterschiedlichen Anzahlen von Bakterien zeigt.

Figur 5 ist eine Graphik des sogenannten "Amplification-Plot", die das Ergebnis der revers transkribierten quantitativen PCR mit einem erfindungsgemäßen Präparat in Beispiel 6 zeigt.

### Detaillierte Offenbarung der Ereindung

Unter Biopartikel werden erfindungsgemäß Mikroorganismen verstanden, insbesondere Bakterien, Viren, Pilze, Protozoen, Bakteriophagen, Hefen, Sporen, Parasiten, Pflanzenzellen, tierische oder menschliche Zellen, Gameten, Plasmide und Viroide. Die Biopartikel enthalten Erbmaterial in Form von Nukleinsäuren. Bevorzugt enthält das erfindungsgemäße Präparat Bakterien, Viren, und/oder Bakteriophagen. Üblicherweise enthält das Präparat eine Art Mikroorganismus, es ist aber auch möglich, dass mehrere Arten gemeinsam vorliegen, z.B. zwei oder mehr Arten Bakterien. Auch eine Kombination von einer Bakterienart und einer Virenart oder einer Bakterienart und einem Bakteriophagen ist z.B. möglich. Weitere Kombinationen sind ebenfalls im Umfang der Erfindung enthalten. Besonders geeignet sind erfindungsgemäß Bakterien und Viren, die bei der Trocknung unter Normalbedingungen ihre Zellwand und Morphologie bewahren. Dies sind z.B. die Bakterien *Corynebacterium glutamicum, Mycobacterium phlei* und *Bacillus subtilis*, die Bakteriophagen Lambda, T7, fr, qβ; MS2 und M13. Das Bakterium *E. coli* ist prinzipiell auch geeignet, wird aber wegen seiner zu großen Instabilität gegen die Lyse (zu leichte Lysierbarkeit) für das erfindungsgemäße Präparat weniger bevorzugt. *C*. *glutamicum* ist besonders geeignet, weil es gut verfügbar und schwer zu lysieren ist, d.h. besonders gut für die Kontrolle der korrekten Lyse verwendbar ist. Der Phage fr ist gut geeignet, weil er ein RNA-Genom aufweist und nur durch reverse Transkription amplifizierbar ist. Damit kann er vorteilhaft für die Kontrolle von Amplifikationen mit reverser Transkription eingesetzt werden.

Unter Normalbedingungen und Umgebungstemperatur wird erfindungsgemäß Raumtemperatur und Normaldruck verstanden, d.h. eine Temperatur von ca. 20-25 °C und ein Druck von ca. 1 bar. Ferner wird darunter eine relative Luftfeuchtigkeit von 20 - 60% verstanden. Umgebungsluft mit einer niedrigeren relativen Luftfeuchtigkeit kann erfindungsgemäß auch benutzt werden.

Das erfindungsgemäße Präparat ist im wesentlichen fest. Dies bedeutet, dass das Präparat bei Normalbedingungen formstabil ist, d.h. nicht zerläuft, zerfällt oder zerfließt. Es kann z.B. in Form von Pellets oder Tabletten vorliegen oder auch in Form von Anhäufungen des Gemisches aus Biopartikeln und Hilfsstoffen, die durch Aufstreichen einer gewissen Menge z.B. auf einer Folie oder Pipettieren in Vertiefungen und anschließendes Trocknen hergestellt werden. In einer alternativen Ausführung kann das Präparat auf einem Stützvlies, z.B. aus Glasfaser hergestellt werden. Die daraus erhaltenen Stanzprodukte stellen das finale Präparat dar.

Insbesondere soll durch den Begriff "im wesentlichen fest" zum Ausdruck gebracht werden, dass es sich nicht um ein flüssiges Präparat handelt, in dem Biopartikel als Suspension in einem Lösungsmittel vorliegen.

Das Präparat enthält weiter übliche pharmazeutische Hilfsstoffe, die zur Herstellung einer festen Zubereitung geeignet sind. Insbesondere werden Fließmittel, Bindemittel, Füllstoffe und/oder Sprengmittel eingesetzt. Diese sind Fachleuten gut bekannt und werden geeignet ausgewählt.

Als Füllstoffe geeignet sind z.B. Stärken wie Mais-, Kartoffel- und Weizenstärke, Lactose, Granulatum simplex (Gemisch aus Kartoffelstärke und Lactose), mikrokristalline Cellulose (z.B. Avicel®), modifizierte Stärken, modifizierte Cellulosen, Glucose, Mannitol, Sorbitol, und Levulose.

Bindemittel sind für die Erhöhung der Festigkeit des festen Präparats bedeutsam. Hierfür geeignet sind z.B. Polyethylenglykole.

Als Fließregulierungsmittel ist z.B. Aerosil® (kolloidale Kieselsäure) geeignet.

Als Sprengmittel sind übliche Sprengmittel wie z.B. Stärke, z.B. Maisstärke oder Kartoffelstärke, Alginate, mikrokristaline Cellulose (Avicel®), Holocellulose (gereinigte ligninfreie Cellulose), Natriumcarboxymethylcellulose, Polyacrylsäure (Carbopol®), Polyvinylpyrrolidon (Polyplasdone®, Kollidon®, Crospovidone®), vernetzte Natriumcarboxymethylcellulose (Ac-Di-Sol®), Natriumcarboxymethylstärke (Primojel®, Explotab®), Natriumcarboxymethylcellulose (Nymcel®) geeignet.

Fließregulierungsmittel, Füllstoffe und Sprengmittel werden geeignet ausgewählt. Bevorzugt werden erfindungsgemäß Hydroxyethylstärke und/oder Carboxymethylcellulose verwendet, weil damit ausreichend feste und stabile Präparate erhalten werden. Als Bindemittel kommt bevorzugt Polyethylenglykol 8000 zum Einsatz.

Dem Präparat können im Verlauf des Herstellungsverfahrens ein oder mehrere Puffer zugesetzt werden. Es werden Puffer verwendet, die stabilisierende und lysierende Eigenschaften für Mikroorganismen haben. Solche Puffer enthalten Detergentien (z.B. SDS (Natriumdodecylsulfat), Lauryl-Sarkosin, Tween 20 (Polyoxyethylensorbitonmonolourat), Triton-X 100 (Polyethylenglykol-p-(1,1,3,3-tetramethylbutyl)-phenylether), Chelatisierungsmittel (z.B. EDTA (Ethylenglykoltetraessigsäure) oder Salze davon wie Na-EDTA (Titriplex)), EGTA (N-(2-Hydroxyethyl)-ethylendiamin), und/oder pH-Pufifiersubsfianzen wie Tris, Hepes (4-(2-Hydroxyethyl)-1-piperazinethansulfonsäure), MOPS (3-(N-Morphoiino)propansulfonsäure), Citrat, Acetat, Ammoniumchlorid). Diese Bestandteile können im verwendeten Puffer einzeln oder als Gemisch vorliegen. Der Puffer wird vom Fachmann gff. geeignet für die Mikroorganismen ausgewählt. Z.B können erfindungsgemäß TE (Tris/Cl pH 8, im Gemisch mit EDTA), oder EDTA in Kombination mit Tris/Cl, Tween 20 und Triton-X 100, Puffer P (1,4 % SDS, 50 mM Titriplex, 500 mM NaCl, 2 % Polyvinylpyrrolidon, 100 mM Na-Acetat-Trihydrat pH 5,4) oder Puffer L (155 mM NH₄Cl, 10 mM KHCO₃) oder Puffer B (50 mM EDTA, 50 mM Tris/Cl pH 8,0, 0,5 % Tween 20, 0,5 % Triton X-1 00) als Puffer verwendet werden. Die in den Puffern enthaltenen Lösungsmittel, insbesondere Wasser, werden zum Abschluss der Herstellung der erfindungsgemäßen Präparate weitgehend vollständig durch Trocknen bei Normalbedingungen entfernt.

Die Mengen der pharmazeutischen Hilfstoffe werden geeignet ausgewählt. Sie können ggf. durch einfache Versuche bestimmt werden. Typischerweise werden z.B. in einem Ansatz 3 g Bindemittel für 1.5 ml Bakterienkulturflüssigkeit oder Bakteriophagen-Überstand eingesetzt. Die Menge des Bindemittels wird ebenfalls geeignet ausgewählt oder durch Versuche bestimmt und beträgt z.B. 0,5 ml 20%-ige Lösung von PEG 8000 für 1,5 ml Bakterienkultur bzw. Bakteriophagen-Überstand. Die Menge der verwendeten Pufferlösung beträgt z.B. 1 ml Pufferlösung für 1 ml Bakterienkultur oder Bakteriophagen-Überstand.

Die Übernacht-Bakterienkultur wird erfindungsgemäß wie folgt erhalten: Die Bakterienkulturen werden als Übernachtkultur heran gezogen. Hierzu werden 20 ml geeignetes Bakteriennährmedium in einen sterilen 100 ml ErlenmeyerKolben pipettiert und mit 5-20 µl Bakteriensuspension angeimpft. Der Kolben wird mit etwas Alufolie locker verschlossen und 10-14 h in einem Schüttelinkubator bei geeigneter Temperatur und Schüttelleistung inkubiert. Nach der Kultivierung wird der Bakterien-Titer eines 1:10 verdünnten Aliquots mit Hilfe einer modifizierten Neubauer Zählkammer durch Auszählen unter dem Mikroskop bestimmt.

Bakteriophagen-Überstände werden durch Infektion von Wirtsbakterien hergestellt. Hierzu werden 20 ml geeignetes Bakteriennährmedium in einen sterilen 100 ml Erlenmeyer Kolben pipettiert und mit 5-20 µl Bakteriensuspension angeimpft. Die Kultur wird 4 h in einem Schüttelinkubator bei geeigneter Temperatur und Schüttelleistung inkubiert. Danach gibt man eine geeignete Menge an Bakteriophagenlösung hinzu und kultiviert für weitere 10-12 h im Schüttelinkubator. Der Fachmann benutzt in der Regel eine MOI (Multiplicity of Infection) von 10, d.h. das Verhältnis von Bakterien zu Bakteriophagen ist 1 : 10. Nach der Kultivierung werden die Bakterien in der Zentrifuge für 10 min bei 8000 Upm sedimentiert und der Überstand wird in ein frisches Zentrifugenröhrchen überführt und ein weiteres Mal zentrifugiert. Der Überstand enthält nun alle freien Bakteriophagen und kann als Aliquots eingefroren werden. Die Bestimmung des Phagentiters (Pfu:
Plaque forming Units) erfolgt nach klassischen Methoden der Mirkobiologie durch Ausplattieren von Verdünnungen des Bakteriophagen-Überstands und
Indikatorbakterien auf Agarplatten. Nach der Inkubation der Agarplatten kann die Anzahl an Pfu durch Auszählen der "Plaques" im "Bakterienrasen" bestimmt werden.

Die geeignete Anzahl an Bakterien und/oder Bakteriophagen, die bei der Herstellung des Präparats zugefügt wird, wird der Fachmann an Hand der finalen Anwendung entscheiden. Sie beträgt z.B. 5.000. Die Mindestanzahl ist etwa 1.000, da bei diesem Wert die Nachweisgrenze liegt. Bevorzugt beträgt sie etwa 10.000 bis etwa 100.000.
Das erfindungsgemäße Präparat kann nach Bedarf geeignete Farbstoffe enthalten, um eine Farbcodierung bereitzustellen. Dies bedeutet, dass erfindungsgemäße feste Präparate je nach dem darin enthaltenen Mikroorganismus unterschiedlich gefärbt vorliegen und so leicht vom Anwender voneinander unterschieden werden können. Hierfür kommen Farbstoffe für histologische Färbungen in Frage, die Fachleuten gut bekannt sind und geeignet ausgewählt werden. Z.B. können Fuchsin, Methylenblau, Eosin, Malachitgrün, Gentianviolett und deren Derivate eingesetzt werden. Die Farbstoffe werden dem Gemisch zur Herstellung des erfindungsgemäßen Präparats entweder als Feststoffe oder in Form einer Lösung zugesetzt, z.B. Wasser und/oder Glycerin und/oder üblichen Alkoholen. Als Beispiele können Gentianviolett Lösung (in Wasser), Fuchsinlösung (in Ethanol), Fosinlösung (in Wasser), Methylenblau-Lösung (in 95% Ethanol) oder Malachitgrün Lösung (in Wasser/Glycerin unter Zusatz von Essigsäure) genannt werden. Derartige Lösungen sind kommerziell erhältlich, z.B. von der Fa. Fluka oder der Fa. Sigma-Aldrich.

Nach Bedarf kann das Präparat auch Antischaummittel enthalten. Diese werden aus den bekannten Antischaummitteln geeignet ausgewählt.

Optional kann das erfindungsgemäße Präparat auch Partikel aus einem harten Material wie Glas, Siliziumcarbid oder Zirkoniumcarbid enthalten. Geeignet hierfür sind auch magnetische Silikapartikel oder Anionaustauscherharz-Partikel. Der Durchmesser dieser Partikel beträgt bevorzugt 100 bis 800 nm. Dieser Zusatz kommt insbesondere in Betracht, wenn das Präparat sehr schwer zu lysierende Mikroorganismen, insbesondere bestimmte Bakterien wie *Corynebacterium glutamicum, Mycobacterium phlei*, Hefe-Pilze; oder Sporen von *Bacillus anthracis* enthält. Diese Ausführungsform kommt insbesondere in Frage, wenn die Lyse, die unter Zusatz des Präparats als interner Standard durchgeführt werden soll, mittels eines Vortex®-Geräts oder in einer Kugelmühle durchgeführt wird. Wie eingangs erwähnt, unterstützen die harten Partikel die Lyse der Mikroorganismen bei diesen Verfahren durch mechanische Einwirkung. Die Menge der harten Partikel wird geeignet ausgewählt und beträgt z.B. 1 0 bis 20 g , bevorzugt etwa 14 bis 1 7 g für 1.5 ml Bakterienkulturflüssigkeit.

Je nach Anwendung kommen Glaspartikel mit unterschiedlichen Durchmessern zur Anwendung:

Das erfindungsgemäße Präparat kann darüber hinaus optional ein oder mehrere Enzyme enthalten. Diese unterstützen ebenfalls die Lyse. Es kommen hierfür Proteasen wie z.B. Proteinase K, Subtilisine; Lipasen und zellwandzersetzende Enzyme wie z.B. Lysozym, Lyticase oder Zymolase in Betracht. Das/die Enzyme und ihre Menge(n) werden geeignet ausgewählt.

Die Herstellung des Präparats kann erfolgen, indem die Bestandteile, die zum Teil auch in flüssiger Form vorliegen, z.B. mit einem Spatel oder auch beliebigen Mischern gemischt und ggf. mit dest. Wasser versetzt werden, bis eine zähe bis dickflüssige homogene Masse entstanden ist. Die einzuarbeitenden Mikroorganismen werden bei dieser Ausführungsform in Form einer Bakterienkulturflüssigkeit oder einer Suspension von Mikroorganismen wie z.B. Phagenüberstand zu den übrigen Bestandteilen gegeben. Die Reihenfolge der Zugabe spielt nur eine Rolle, wenn das Präparat noch zusätzlich noch harte Partikel enthalten soll. Allzulanges Rühren kann die Bakterien in diesem Fall bereits durch Mahleffekte lysieren, daher wird die Suspension von Mikroorganismen in diesem Fall als allerletzte Zutat beigefügt und auch nur sehr vorsichtig mit den übrigen Bestandteilen vermischt. Ansonsten spielt die Reihenfolge der Zugabe und Vermischung der einzelnen Bestandteile keine Rolle.

Die erhaltene dickflüssige Masse wird z.B. in eine Spritze überführt und mit einer stumpfen Kanüle (z.B. als kleine Kügelchen (Durchmesser z.B. 1-2 mm)) auf eine geeignete Folie wie z.B. Parafilm oder Polyethylenfolie oder Teflonfolie portionsweise aufgetragen und anschließend bei Normalbedingungen getrocknet. Auch mit einer anderen geeigneten üblichen Portioniervorrichtung kann das Portionieren erfolgen. Alternativ kann die dickflüssige Masse auch in geeignete Formen wie z.B. Tablettenblister gefüllt und dann bei Normalbedingungen getrocknet werden. Weiterhin kann ein festes Präparat durch Auftragen der dickflüssigen Masse auf einem Stützvlies, z.B. aus Glasfaser, Trocknen und Ausstanzen von beliebigen Formen erfolgen. Üblicherweise erfolgt das Trocknen während mehrerer Stunden, z.B. über Nacht, bevorzugt im Abzug. Überraschenderweise ist weder Kühlen noch Arbeiten unter Schutzgas erforderlich.

In einer weiteren Ausführungsform werden alle Bestandteile des festen Präparats mit Ausnahme der Mikroorganismen wie vorstehend gemischt, portioniert und getrocknet. Dann wird nachträglich eine Lösung oder Suspension der Mikroorganismen auf die erhaltenen festen Präparate, z.B. mittels einer Pipette, aufgetragen, und anschließend wird nochmals unter den vorstehend genannten Bedingungen getrocknet. Hierdurch erfolgt gewissermaßen ein Imprägnieren des festen Präparats mit den Mikroorganismen. Überraschenderweise wird auch auf diese Art und Weise ein stabiles festes Mikroorganismen enfhaltendes Präparat erhalten. Diese Vorgehensweise ist sowohl für Bakterien als auch für Phagen besonders geeignet. Sie bietet den Vorteil, dass man die Mengen der auf jede Tablette aufgebrachten Mikroorganismen durch Titerbestimmung nach einem üblichen Verfahren und anschließende Grenzverdünnung genau festlegen kann.

Die erhaltenen erfindungsgemäßen festen Präparate sind unter Normalbedingungen mehrere Wochen stabil. Dies bedeutet, dass bei Resuspendieren des bei Normalbedingungen gelagerten Präparats in Wasser oder wässriger Pufferlösung nach mehreren Wochen noch eine ausreichende Anzahl lebender Mikroorganismen vorliegt, um eine Verwendung als interner Standard in Nukleinsäurepräparations- und Nukleinsäurenachweisverfahren zu ermöglichen.

Dies ist sehr überraschend, da man bisher davon ausging, dass derartig stabile Mikroorganismen-Präparate nur durch Lyophilisierung erhalten werden könnten und ausschließlich unter Zusatz von Zuckern wie z.B. Trehalose oder Mannitol stabil seien.

Das erfindungsgemäße feste Präparat ist leicht herzustellen, ohne dass aufwendige und teure Verfahren wie Lyophilisierung erforderlich sind und dass Zusätze wie die genannten Zucker notwendig sind.

Das Präparat ist leicht zu handhaben, da der Anwender es einfach der zu analysierenden Probe, wie z.B. Blut, Liquor o.ä. zugeben kann. Dadurch werden gleichzeitig der Kontakt mit den im Präparat enthaltenen Mikroorganismen und Verluste von Material sowie Kontamination von Geräten und Laborumgebung, die bei Verwendung von Suspensionen bzw. Flüssigkeiten zwangsläufig auftreten, vermieden.

Besonders einfach wird die Handhabung des erfindungsgemäßen Präparats, wenn es bereits in mikrofluidischen Vorrichtungen oder LoCs enthalten ist, so dass der Anwender gar nicht mehr direkt mit dem Präparat in Berührung kommt und gar nicht erst die Möglichkeit besteht, dass der interne Standard vergessen wird.

Die erfindungsgemäßen Präparate werden als interne Standards in Nukleinsäurepräparations- und Nukleinsäurenachweisverfahren eingesetzt. D.h., sie werden in üblichen Verfahren zur Nukleinsäurepräparation und zum Nukleinsäurenachweis vor oder beim ersten Reaktionsschritt oder auch erst nach der Präparation der Nachweismischung zugesetzt.

Besonders bevorzugt kann ihr Einsatz in mikrofluidischen Präparations- und Nachweis-Verfahren erfolgen, wo sie der zu präparierenden Probe vor dem Aufgeben auf die mikrofluidische Vorrichtung zugesetzt werden können. Alternativ ist es auch möglich, das erfindungsgemäße Präparat in der mikrofluidischen Vorrichtung so vorzusehen, dass es bei der Aufgabe der zu präparierenden Probe in dieser suspendiert und dann mit ihr zusammen weiter präpariert wird, d.h. als Bestandteil eines LoC oder einer Nachweis-Cartridge.

Das erfindungsgemäße feste Präparat kann für jedes übliche Aufschluss- bzw. Lyseverfahren wie vorstehend beschrieben als interner Standard verwendet werden. Die Probe wird dazu gemeinsam mit dem erfindungsgemäßen Präparat und einem geeigneten Lysepuffer gemischt. Anschließend wird das Gemisch durch Wärme, Ultraschall oder "bead beating" behandelt. Der Lysepuffer ist z.B. Buffer AL (Fa QIAGEN GmbH; Hilden, Germany). Weitere Lysepuffer sind Fachleuten bekannt und werden passend für den zu lysierenden Mikroorganismus ausgewählt, ebenso wie die geeigneten Lysebedingungen. Es können auch mehrere verschiedene Lysepuffer eingesetzt werden. Z. B. kann als Lysepuffer PBS-Puffer (phosphatgepufferte Kochsalzlösung) verwendet werden, der Proteinase K und Lysozym enthält. Gegebenfalls wird zunächst inkubiert und dann ggf. ein weiterer Puffer zugegeben wie z. B. Puffer G (GITC (Guanidiniumthiocyanat)/Nonidet® (Nonylphenylethylenglykol)) und nochmals inkubiert. Das geeignete Volumen des/der Puffer muss in Abhängigkeit zum Probenmaterial bestimmt werden und beträgt z.B. je nach Probenmaterial zwischen 50 µl und 1 ml. Die Temperatur und Zeit zur Inkubation wird jeweils geeignet ausgewählt. Da manche Lyseverfahren mittels Einsatz von Enzymen erfolgen, ist die gewählte Temperatur auch abhängig von der Aktivität der Enzyme. Zymolase, Lyticase und Lysozym werden in der Regel im Temperaturbereich von 20°C - 37°C eingesetzt. Die Inkubation mit Proteasen , z.B. Proteinase K oder Subtilisin, erfolgt in der Regel bei 50 - 60 °C. Die Dauer der Inkubation beträgt in der Regel 5 bis 20 Minuten, bevorzugt ca. 10 Minuten. Die Inkubation kann z.B. in einem Thermomixer durch Schütteln erfolgen, z.B. bei ca. 1400 Upm, oder auch auf einem Vortex®-Gerät (maximale Leistungsstufe)), ggf. unter Zusatz von Glasperlen, wobei das erfindungsgemäße Präparat selbst bereits Partikel aus einem harten Material und/oder Enzyme zur Unterstützung der Lyse enthalten kann.

Im Anschluss an die Lyse erfolgt die Isolierung der durch die Lyse freigesetzten Nukleinsäuren durch beliebige im Stand der Technik bekannte Reinigungsverfahren. Hierfür kann der erhaltenen Mischung ein geeignetes Lösungsmittel zugesetzt werden, z.B. Ethanol. Die Reinigung der in dem Lysat enthaltenen Nukleinsäuren erfolgt im allgemeinen durch Adsorption.

Bei den Reinigungsverfahren durch Adsorption nutzt man das selektive Binden der Bestandteile des Inhalts des Lysats an oder auf einem festen Stütz- oder Trägermaterial ("Binden"), das Beseitigen unerwünschter Bestandteile vom festen Stütz- oder Trägermaterial ("Waschen"), und das Lösens des gewünschten Bestandteils ("Elution"),

Um ein gewünschtes Absorbieren und Desorbieren während der Aufreinigung der Biomoleküle zuzulassen, sind spezielle Filterelemente entwickelt worden, die z.B. aus Silika-Gel gebildet sind, und die einerseits porös oder Matrixartig sind, um eine Flüssigkeit durch das Filterelement hindurch gelangen zu lassen, und die andererseits eine Oberfläche haben, an die die Biomoleküle in einem spezifischen oder unspezifischen Prozess binden. In anderen Reinigungsverfahren werden Biomoleküle auf Filterelementen einfach durch den Effekt des Größenausschlusses zurückgehalten. Wenn eine Flüssigkeit, die ein Biomolekül wie z.B. eine Nukleinsäure enthält, durch das Filterelement hindurch tritt, bleiben in jedem Fall die Biomokeküle oder ein Teil davon in dem Filterelement, während der Rest durch das Filterelement hindurch gelangt. Des Weiteren wird, um das Biomolekül aus dem Filterelement zu gewinnen, eine Elutionsflüssigkeit, z.B. Nuklease-freies Wasser, zum Desorbieren des Biomoleküls auf das Filterelement geführt. Auf diese Weise wird das gewünschte Biomolekül von dem Filterelement gelöst (eluiert), und in einem Gefäß aufgefangen. Solche Filterelemente werden häufig als Membranen ausgelegt, die entweder in einzelnen Gefäßen angeordnet sind, die eine Eingangsöffnung und eine Ausgangsöffnung haben, oder die in Multiwell-Platten angeordnet sind. Die Filterelemente werden entweder mit Zentrifugen ("spin format"), oder mit auf VakuumTechnologie basierenden Apparaten prozessiert. Einzelne Gefäße mit einer Eingangsöffnung und einer Ausgangsöffnung, die eine Membran haben und die in einer Zentrifuge benutzt werden können, sind auch als Säulen, Zentifugensäul(ch)en, Filtergefäße, Chromatographiesäulen, "columns", "spin columns" oder "single spin columns" bekannt.

Eine weitere Ausführungsform der Adsorptivmedien zur Nukleinsäure-Isolierung stellen magnetische Silika-Partikel dar. Prinzipiell sind die in der Erfindung vorgestellten Kontroll-Präparate auch kompatibel mit diesen Methoden der Nukleinsäure-Isolierung.

Die Firma QIAGEN GmbH aus Hilden, Deutschland, bietet ein breites Spektrum an Reinigungsprotokollen und an dafür benötigten Produkten für unterschiedliche Biomoleküle aus einer Vielzahl biologischer Proben an, die auf dem Grundprinzip des "Bind-Wash-Elute"-Protokolls basieren. Im Handel ist beispielweise das Produkt "QIAGEN OIAprep Spin Miniprep Kit" erhältlich, Zu diesem Zweck werden unterschiedliche Filtermaterialien und - vorrichtungen verwendet, wie beispielsweise beschrieben in WO 03/040364 oder US 6,277,648.

Bekannt ist also zur Prozessierung einer Probe ein Gefäß zu verwenden, welches oben offen und verschließbar ist. Am Grund des Gefäßes befindet sich eine Membran als Filterelement. Unterhalb der Membran befindet sich eine Öffnung in Form eines Stutzens, die mit beispielsweise einem Schlauch verbunden ist. Über diesen Schlauch kann Flüssigkeit aus dem Gefäß abgesaugt werden.

Zur Prozessierung wird die biologische Probe oben in das Gefäß hineingegeben. Anschließend wird die jeweils vorgesehen Lösung hineingegeben, also z. B. zunächst ein Lysepuffer, um die Probe aufzuschließen. Ist die Probe aufgeschlossen worden, so wird der Lysepuffer nach unten aus dem Gefäß herausgesaugt. Das Lysat enthält chemisch/physikalische Bedingungen, die eine Bindung der Nukleinsäure an das Trägermaterial bewirken. Die aufgeschlossene Probe (Lysat) wird anschließend gewaschen und eluiert.

Nachdem die Nukleinsäure der aufgeschlossenen Probe an die Membran gebunden oder adsorbiert wurde, werden Waschpuffer von oben in das Gefäß gegeben und nach unten aus dem Gefäß abgesaugt oder zentrifugiert. Der Waschpuffer erhält diese Bindung aufrecht, während er gleichzeitig ungewünschte Zellbestandteile aus der Membran wäscht.

Waschpuffer enthalten in der Regel Ethanol. Ethanol bewirkt eine Bindung von Nukleinsäure unter bestimmten Voraussetzungen an eine als Filterelement wirkende Membran. Eine als Filter dienende Membran weist ein gewisses Totvolumen von beispielsweise 40 µl auf. Es kann daher nicht verhindert werden, dass stets eine entsprechende Menge an Ethanol im Filter verbleibt. Ethanol trägt einerseits unerwünscht zur Bindung bei.

Andererseits kann Ethanol aber auch das Ergebnis einer späteren Analyse verfälschen. Um die Nukleinsäure aus dem Gefäß nach unten entnehmen zu können und fehlerhafte Analyseergebnisse zu vermeiden, wird zunächst Ethanol entfernt, beispielsweise, indem das Gefäß zentrifugiert wird. Das Ethanol entweicht so aus der Membran und kann nach unten entnommen werden. Im Anschluss daran ist die Membran hinreichend frei von Ethanol. Mittels Vakuum und Wärmezufuhr kann Ethanol aus der Membran alternativ entfernt werden.

Um die Nukleinsäure wieder von der Membran zu lösen, wird eluiert. Dies geschieht regelmäßig mit Wasser oder einer schwach salzhaltigen wässrigen pH-stabilisierten Lösung. Im Anschluss an die Elution kann die Nukleinsäuren durch die Membran hindurch aus dem Gefäß nach unten hin entnommen werden.

Alternativ kann auch Adsorption an Magnetpartikeln und anschließendes Waschen und Elution erfolgen.

Die geeigneten Adsorptionsmaterialien, die Waschpuffer und die Elutionspuffer sind Fachleuten bekannt und werden geeignet ausgewählt.

In vorteilhafter Weise kann das Adsorbieren/Waschen/Eluieren bei der Präparation von DNA z.B. gemäß dem "QIAamp DNA Micro Handbook" (www1.qiagen.com/HB/QIAampDNAMicro) auf einer QIAamp MinElute Spin Column erfolgen, die von der Fa. Qiagen angeboten wird. Nach der Bindung wird zentrifugiert und anschließend mit einem oder ggf. mehreren geeigneten Puffern gewaschen, z.B. mit Buffer AWI (GuanidiniumHydrochlorid, Ethanol), Buffer AW2 (NaCl, Tris/Cl pH 7,5, Ethanol), wobei üblicherweise nach jedem Waschschritt erneut zentrifugiert wird, um die Waschpuffer zu entfernen. Für die anschließende Elution der Nukleinsäuren kann z.B. der Puffer TE (10 mM Tris/Cl pH 8,0; 1 mM EDTA) eingesetzt werden. Üblicherweise wir nach der Elution ebenfalls zentrifugiert, um die Nukleinsäuren vollständig von der Membran zu entfernen.

Alternativ kann auch für die RNA-Präparation z.B. eine RNeasy Spin Column der Fa. QIAGEN verwendet und gemäß dem RNeasy Mini Handbook (www1.qiagen.com/HB/RNeasyMini) verfahren werden. In diesem Fall können z.B. die Waschpuffer Buffer RW1 (Guanidiniumisocyanat, Methanol) und RPE (Tris/Cl pH 7,5, Ethanol) und zur Elution RNAse-freies Wasser verwendet werden, wobei nach jedem Wasch-/Elutionsschritt ebenfalls vorteilhafterweise eluiert wird.

Bei Durchführung des Präparations- und Nachweisverfahrens in mikrofluidischen Vorrichtungen werden als Waschpuffer bevorzugt Buffer AW1 oder Buffer RW1 bzw. Buffer AW2 oder Buffer RPE benutzt, und als Elutionspuffer bevorzugt RNAse-freies Wasser verwendet. Das Zentrifugieren nach jedem Wasch- bzw. Elutionsschritt entfällt hierbei.

Als Nukleinsäurenachweisverfahren ist ebenfalls jedes übliche Verfahren für die Anwendung des erfindungsgemäßen Präparats geeignet. Bevorzugt wird das erfindungsgemäße Präparat in PCR-Nachweisverfahren eingesetzt, besonders bevorzugt in Duplex-Realtime PCR-Verfahren wie eingangs beschrieben. Für die Erläuterung der PCR wird auf die eingangs gegebene Erläuterung verweisen.

Die Wahl des im erfindungsgemäßen Präparat vorliegenden Mikroorganismus bestimmt, welcher Typ Nukleinsäure in der zu analysierenden Probe nachgewiesen werden kann. Bei Verwendung von Bakterien können sowohl RNA als auch DNA nachgewiesen werden, bevorzugt ist DNA. RNA-Nachweise aus Bakterien sind zwar grundsätzlich möglich, aber nicht sinnvoll, da nicht nur die RNA spezifisch amplifiziert wird, sondern auch die DNA. Bei Verwendung von Bakteriophagen und Viren bestimmt die Art der Nukleinsäure des Genoms im Viruspartikel, ob RNA oder DNA in der Probe nachgewiesen wird. Bei Verwendung von Protozoen oder Hefepilzen als Kontroll-Mikroorganismus kann nicht nur deren chromosomale DNA nachgewiesen werden, sondern auch eine selektive Amplifikation von RNA ist möglich, da dies Organismen über Mechanismen des mRNA-Splicing verfügen.

Für die Durchführung der PCR sind alle gängigen Reagenzien und dafür verfügbare Kits einsetzbar. Die Primer, Desoxynukleotid-Triphosphat, Polymerase, die Additive, Cosolventien und Markierungs-Sonden sind, wie eingangs beschrieben, dem Fachmann bekannt und werden geeignet ausgewählt. Beispielsweise können für eine TaqMan®-Analyse entsprechende PCR-Reagentiensätze von QIAGEN, wie der QuantiTect Probe PCR Mastermix, der QuantiTect Probe RT-PCR MasterMix, bzw. der QuantTect RT Mix verwendet werden.

Als Primer können z.B. "fr Forward" (CTTCTGATCCGCATAGTGACGAC) (SEQ ID NO:7), "fr Reverse" (AACGGTCATTCGCCTCCAGCAG) (SEQ ID NO:8) und "fr Probe" (,6-FAM'-TAGGGGATGGTAACGACGAAGCA-,BHQ1a') (SEQ ID NO:9) eingesetzt werden, in dieser Kombination besonders vorteilhaft, wenn als Mikroorganismus der Phage fr eingesetzt wird.

Insbesondere bei Verwendung von Corynebacterium *glutamicum* als Mikroorganismus wird in vorteilhafter Weise die Kombination von CG Forward (AAGCTCCAGCCACCCAAACTAC) (SEQ ID NO: 1), CG Reverse (CTACCAACCACTAATGCGTCATC) (SEQ ID NO:2) und CG Probe (,6-FAM'-ATCGCCTTCCAGACGCTCAACG-,BHQ1a') (SEQ ID NO:3) eingesetzt.

Wird *Bacillus subtilis* als Kontroll-Mikroorganismus verwendet, so kann dieser mit folgender Primer-Kombination nachgewiesen werden: BS Forward (ACATCTTACCGCAACTACGACCAT) (SEQ ID NO:4), BS Reverse (TAGCATAGTCTTTGTCCCACCGTA) (SEQ ID NO:5) und BS Probe (,6-FAM'-GGAGGCGATCTATGTCTTGTCCA -,BHQ1a') (SEQ ID NO:6).

Die Primer können mit Fachleuten bekannten Verfahren synthetisch hergestellt werden oder sind kommerziell bei den Firmen Operon oder MWG Biotech erhältlich.

Als Marker können alle Marker verwendet werden, die Fachleuten bekannt sind. Insbesondere werden zur Detektion der Kontroll-Mikroorganismen Fluoreszenzmarker verwendet, besonders vorteilhaft die Kombination von 6-FAM am 5' Ende des Oligonukleotid und "Black Hole Quencher" (BHQ) am 3'-Ende. Für die Detektion der in der Probe nachzuweisenden Mikroorganismen werden ebenfalls geeignete Marker z.B "HEX" ausgewählt, die sich von den genannten unterscheiden. Der Fachmann wird je nach Bedarf auch andere Fluorophore einsetzen.

Als Lösungsmittel für die PCR Reagenzien und Primer wird üblicherweise Wasser verwendet.

Zur PCR werden weiterhin Templat-Aliquots in Form des Eluats zugegeben, die die zu amplifizierenden Nukleinsäuren enthalten.

Die Temperaturzyklen der PCR werden vom Fachmann je nach der zugrundeliegenden Ausgangsnukleinsäure geeignet ausgewählt und können z.B. 15 Min bei 95°C und 40 x [15 Sekunden bei 95°C, 1 Minute bei 60°C] sein oder für eine RNA-basierende Amplifikation 30 Minuten bei 50°C (Reverse Transkription), 15 Minuten bei 95°C und 40 x [1 5 Sekunden bei 95°C und 1 Minute bei 60°C] betragen. Die Kontrolle der Temperatur und das Erwärmen und Abkühlen erfolgen auf übliche Weise.

Der Nachweis der Amplifizierungsprodukte kann je nach den verwendeten Markern wie eingangs beschrieben auf unterschiedliche Weise erfolgen. Dies ist dem Fachmann bekannt. Bei Verwendung von Fluoreszenzmarkern erfolgt die Detektion fluoreszenzspektrometrisch. In üblichen PCR-Verfahren können bis zu 6 verschiedene Fluorophore gleichzeitig nachgewiesen werden (6-Kanal-Multiplexing). Es wird dann die Anzahl von PCR-Cyclen bestimmt, ab der ein deutlicher Anstieg der Fluoreszenz des spezifischen Markers auftritt (Ct, threshold cycle).

Beobachtet man bei diesem Nachweis sowohl Amplifizierungsprodukte der Nukleinsäuren aus dem Kontroll-Mikroorganismus als auch aus der zu analysierenden Probe, so kann man davon ausgehen, dass sowohl die Präparation als auch die Amplifikation für beide korrekt verlaufen sind und dass der Nachweis des zu analysierenden Mikroorganismus positiv ist.

Beobachtet man nur Amplifizierungsprodukte von Nukleinsäuren des nachzuweisenden Mikroorganismus, aber nicht des Kontroll-Mikroorganismus, oder aber gar keine Amplifizierungsprodukte, war die Präparation und/oder die Amplifikation nicht korrekt und muss wiederholt werden.

Beobachtet man nur Amplifizierungsprodukte der Nukleinsäuren des Kontroll-Mikroorganismus, aber keine des nachzuweisenden Mikroorganismus, war die Präparation und Amplifikation korrekt, und das Testergebnis ist negativ.

Die folgenden Beispiele beschreiben die Erfindung näher.

Die in den Beispielen verwendeten Mikrorganismen sind kommerziell bei der American Type Culture Collection, Manassas, VA, USA, erhältlich. Es handelt sich um *Bacillus subtilis* ATCC 23857, *Corynebacterium glutamicum* ATCC 13032 und E. coli Phage fr ATCC 15767-B1.

Die Begriffe "Standardabweichung", "Mittelwert" und "Variationskoeffizient (CV)" werden im folgenden wie übliche verwendet und mit üblichen statistischen Methoden ermittelt. Für diagnostische Proben sollte ein CV geringer sein als 8%.

### Beispiel 1: Herstellung von Tabletten

### a) Tablette A (Corynebacterium glutamicum)

3 g Hydroxyethylstärke, 0,5 ml 20 % PEG-8000, 1,5 ml TE (10 mM Tris-Cl pH 8,0; 1 mN EDTA), 1.5 ml Übernacht-Kultur von *Corynebactrium glutamicum* (erhalten wie vorstehend beschrieben) in 2 YT-Medium (yeast extract tryptone medium, Hefeextrakt-Trypton-Medium)) und weinige Krümel Fuchsin (Fa. Fluka) werden in eine Einmal-Wägeschale gegeben und mit einem Spatel zu einer homogenen Masse vermischt. Die Masse wird mit dem Spatel in eine Spritze überführt und über eine stumpfe Kanüle als kleine Kügelchen (Durchmesser 1-2 mm) auf Parafilmfolie portioniert. Anschließend werden die erhaltenen Tabletten über Nacht im Abzug getrocknet. Man erhält auf diese Weise je nach verwendeten Aliquots 50 bis 200 Tabletten.

### b) Tablette B (Coryrrebacterium glutamicum)

1 g Hydroxyethylstärke, 500 µl 20 % PEG-8000, 500 µl Übernacht-Kultur von *Corynebacterium glutamicum* in 2YT-Medium (erhalten wie vorstehend beschrieben), 1 kleine Spatelspitze Methylenblau, 750 µl Puffer P (1,4 % SDS, 50 mM Titriplex, 500 mM NaCl, 2 % Polyvinylpyrrolidon, 100 mM Na-Acetat-Trihydrat pH 5,4) 500 µl Puffer L (155 mM NH₄Cl, 10 mM KHCO₃, und 50 mg Carboxymethylcellulose werden wie bei Tablette A gemischt, auf Folie portioniert und über Nacht getrocknet.

### c) Tablette C (Phage fr)

3 g Hydroxyethylstärke, 150 mg Carboxymethylcellulose, 1 ml 20 % PEG-8000, 1 ml Puffer B (50 mM EDTA, 50 mM Tris/Cl pH 8,0, 0,5 % Tween 20, 0,5 % Triton X-100), 1 ml bidest. Wasser, 1 kleine Spatelspitze Eosin und 1 ml Phagenüberstand des Phagen fr (erhalten wie vorstehend beschrieben) werden wie bei Tablette A gemischt, portioniert und über Nacht getrocknet.

### d) Tablette D (Phage fr)

Die Tablette wurde genauso wie Tablette C hergestellt mit der Ausnahme, dass 1 ml Pagenüberstandslösung durch 1 ml bidest. Wasser ersetzt wurde. Nach dem Trocknen wurden je 4 µl Phagenüberstandslösung (erhalten wie vorstehend beschrieben) auf jede getrocknete Tablette pipettiert und erneut über Nacht im Abzug nachgetrocknet.

### e) Tablette E (Bakterien mit Glasperlen):

0,75 g Hydroxyethylstärke, 150 mg Polyvinylalkohol 30.000-70.000, 150 mg Polyvinylpyrollidon K15 MG∼10.000 und 15,9 g Glassbeads 425-600 µm (Sigma-Aldrich G8772-500G) werden in einer Wägeschale abgewogen. Nach Zugabe von 1,5 ml Puffer B [50 mM EDTA; 50 mM TrisCl pH 8.0; 0.5 % Tween 20; 0.5 % Triton X-100], 1,5 ml 20% PEG 8000, 20 µl Malachitgrün-Lösung (Fluka) sowie 90 µl Wacker Chemie-Antischaummittel auf Silikon-Basis wird durch sorgfältiges Rühren eine homogene Suspension hergestellt. Nun wird in diese Masse 1,5 ml Bakterienkulturflüssigkeit (erhalten wie vorstehend beschrieben) ganz vorsichtig eingerührt. Aliquots dieser Masse werden dann auf einer geeigneten Grundlage getrocknet.

### f) Tablette F (Bakterien mit Glasperlen)

1,5 g Hydroxyethylstärke, 50mg CMC (Carboxymethylcellulose), und 8 g Glassbeads 100 µm (Sigma-Aldrich G4649-500G) werden in einer Wägeschale abgewogen. Nach Zugabe von 0,5 ml Puffer B [50 mM EDTA; 50 mM TrisCl pH 8.0; 0.5 % Tween 20; 0.5 % Triton X-100], 0,5 ml 20% PEG 8000, 1,25 ml bidest. Wasser und 1 Tropfen Gentianviolett-Lösung (Fluka) wird durch sorgfältiges Rühren eine homogene Suspension erstellt. Nun wird in diese Masse 0,75 ml Bakterienkulturflüssigkeit (erhalten wie vorstehend beschrieben) ganz vorsichtig eingerührt. Aliquots dieser Masse werden dann auf einer geeigneten Grundlage getrocknet.

### Beispiel 2 (Nukleinsäure-Präparation und -Amplifikation):

### a) Lyse/Präparation

Je eine Tablette A wurde in ein 2 ml Röhrchen gegeben und mit 200 µl Blut (Beispiel 2a) bzw. 200 µl PBS-Puffer (Beispiel 2b) versetzt. Zu jedem Röhrchen wurden je 20 µl QIAGEN Proteinase K Lösung und 40 µl Lysozym-Lösung (je 20 mg/ml in Wasser) gegeben. Die Inkubation erfolgte während 10 Minuten bei 56 °C auf einem Eppendorf-Thermomixer bei 1400 Upm Schüttelgeschwindigkeit. Anschließend wurden 200 µl Puffer G (3 M GITC (Guanidiniumthiocyanat), 20 % Nonidet® P40) zugegeben und erneut bei 56°C währende 10 Minuten auf einem Eppendorf-Thermomixer bei 1400 Upm Schüttelgeschwindigkeit inkubiert. Dann wurden 200 µl Ethanol zugegeben und gut mit dem Röhrcheninhalt vermischt.

### b) Reinigung

Folgende Schritte wurden anschließend mit jeder der beiden Proben gemäß dem "QIAmp DNA Micro Handbook" durchgeführt:
- Bindung:: Das gesamte Gemisch wurde auf auf QIAmp MinElute Spin Column geben und 1 Minute bei 6000 x g zentrifugiert Waschen: mit 500 µl Puffer AW1 waschen, bei 6000 x g 1 Minute zentrifugieren, 500 µl Puffer AW2 zugeben, 3 Minuten bei maximaler g-Zahl zentrifugieren
- Trocknen:: "Dry Spin" (Trocknungschritt der Spin Column Membran in der Zentrifuge in Abwesennheit von jeglichen Waschpuffer-Resten) für 1 Minute bei maximaler g-Zahl, um alles Lösungsmittel zu entfernen
- Elution:: mit 80 µl Puffer TE (10 mM Tris/Cl pH 8,0, 1 mM EDTA) eluieren, 1 Minute bei 6000 x g zentrifugieren

Dasselbe Verfahren wurde für Tablette B durchgeführt.

### c) Mengenbestimmung durch UV Quantifizierung und AgaroseGelelektrophorese

Die Mengenbestimmung der Nukleinsäuren erfolgte bei allen erhaltenen Eluaten (Tablette A und B, jeweils in Blut und PBS) mittels Messung der optischen Dichte bei 260 nm am Spektralphotometer. Die optische Dichte wurde jeweils mit einer Wellenlänge des Lichts von 260 nm ermittelt. Die optische Dichte ist ein Maß für die Menge der im Eluat vorliegenden DNA. 1 OD entspricht 50 mg/ml doppelsträngiger DNA. Aus der optischen Dichte kann damit (bei Verwendung einer geeigneten Verdünnung) die Konzentration der im Eluat der Probe vorhandenen DNA berechnet werden.

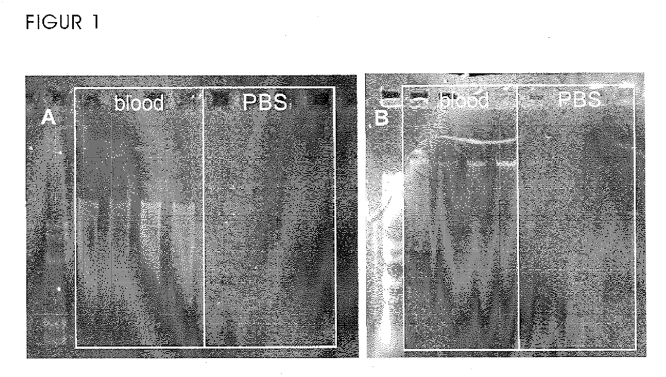

Nach der Präparation gemäß den vorbeschriebenen Protokollen wurden die Nukleinsäuren auf einem Agarosegel elektrophoretisch aufgetrennt. Es wurde eine Lösung von 0,7% w/v Agarose bis zum Schmelzen der Agarosepartikel erhitzt, und nach der Homogenisierung ließ man sie in einer Gelform erstarren. Das Gel enthielt Taschen, in die die Nukleinsäure-haltige Lösung pipettiert und elektrophoretisch aufgetrennt wurde. In diesem Fall wurden 15 µl des Eluats nach Nukleinsäure Präparation aufgetragen, wobei in Figur 1 der Bildausschnitt -A: die Ergebnisse der Tablette A und -B: die Ergebnisse der Tablette B darstellt.

Nach der Auftrennung wurden die Nukleinsäurefragmente in einem Ethidiumbromid-haltigen Wasserbad angefärbt, auf einer UV-Leuchtplatte zum Leuchten gebracht und durch ein Fotodokumentationssystem abgelichtet. Die genomische DNA ist als helle Bande im oberen Drittel der linken Seite (Blut) von Figur 1A bzw. der unteren Hälfte der linken Seite (Blut) der Fig. 1A zu sehen.

Es ist deutlich erkennbar, dass nur in den Blut-Proben genomische DNA (aus den weißen Blutkörperchen) im Gel sichtbar ist, während in den PBS-Proben laut Gel keine DNA vorliegt. Dies zeigt, dass die in den erfindungsgemäßen Präparaten A und B eingesetzte Menge *Corynebacterium glutamicum* zu gering ist, um in der Agarosegelelektrophorese nachgewiesen zu werden.

Die Ergebnisse der Quantifizierung der Optischen Dichte der Eluate der in Beispiel 2 erhaltenen Mengen an Nukleinsäuren (vor der PCR) sind in der folgenden Tabelle 1 dargestellt:

**Tabelle 1**

| Probe | Tablette A | | Tablette B | |
|---|---|---|---|---|
| | Konz. (ng/µl) | Ausbeute (µg) | Konz. (ng/µl) | Ausbeute (µg) |
| Blut | 23,5 | 1,88 | 14,7 | 1,18 |
| Blut | 30,4 | 2,44 | 6,4 | 0,51 |
| Blut | 34,7 | 2,78 | 22,4 | 1,79 |
| Blut | 25,3 | 2,03 | 25,9 | 2,07 |
| PBS | 4,3 | 0,35 | 5,9 | 0,47 |
| PBS | 5,5 | 0,44 | 9,7 | 0,78 |
| PBS | 6,2 | 0,50 | 14,1 | 1,13 |
| PBS | 6,4 | 0,51 | 10,8 | 0,86 |

In den Blut-Proben wurden jeweils ca. 20-30 ng/µl DNA gefunden (aus weißen Blutkörperchen), während in den PBS-Proben nur ca. 5-6 ng/µl DNA vorlagen, die aus dem im Präparat eingesetzten *C. glutamicum* stammten. Während also die Gelelektrophorese nicht empfindlich genug ist, um die geringen Mengen Kontroll-Mikroorgansimus nachzuweisen, reicht die Empfindlichkeit der photometrischen Bestimmung dafür aus.

### Beispiel 3: Quantitative PCR (spezifisch für C. glutamicum)

Die für *C. glutamicum* spezifische Real Time PCR wurde mit 10.0 µl QIAGEN QuantiTect Probe PCR Mastermix, 0, µl CG Forward (SEQ ID NO:1) (100 µM), 0,10 µl CG Reverse (SEQ ID NO:2) (100µM), 0,05 µl CG Probe (SEQ ID NO:3) (100 µM), 7,75 µl bidest. Wasser und 2,0 µl Templat-Aliquots der in Beispiel 2 mit Blut- bzw. PBS-Proben jeweils mit Tablette A und B erhaltenen Eluate durchgeführt. Die Temperaturzyklen waren 15 Minuten bei 95 °C und 40 x (15 Sekunden 95 °C, 1 Minute bei 60 °C). Die Amplifikation wurde jeweils mit Eluaten von 4 einzelnen Nukleinsäurepräparationen durchgeführt.

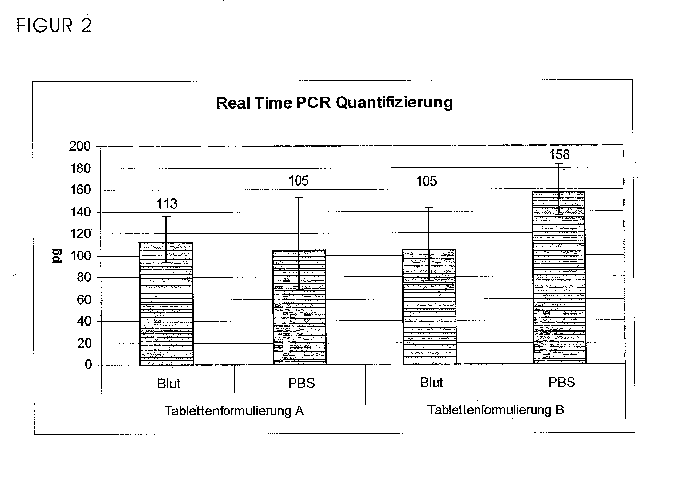

Die Ergebnisse sind in Figur 2 dargestellt. Das Histogramm zeigt die durch die PCR nachgewesene Menge *C*. *glutamicum* DNA (in pg) pro Reaktion bei einer Stichprobenmenge von 4. Es wurden jeweils die Ct-Werte in der RT-PCR fluoreszenzspektroskopisch ermittelt. Mit Hilfe einer Eichkurve wurde die jeweilige Anfangskonzentration der DNA im jeweiligen Eluat ermittelt. Die Werte der 4 unterschiedlichen Ansätze und Präparationen liegen innerhalb einer Standardabweichung. Die Menge an *C*. *glutamicum* ist in allen Proben und Ansätzen etwa gleich. Dies zeigt, dass die erfindungsgemäßen Präparate in reproduzierbarer Weise für die Kontrolle sowohl der Präparation von Nukleinsäuren als auch der PCR eingesetzt werden können.

### Beispiel 4: Duplex-gPCR

Präparate von *C. glutamicum* wurden nach der Tabletten-Rezeptur B (Beispiel 1 b) hergestellt. In drei unabhängigen Bestimmungen wurde jeweils 1 Präparat mit 200 µl Vollblut versetzt. Nach dem Protokoll des Beispiels 2 wurden die Nukleinsäuren präpariert, und es wurde jeweils mit 2 µl Eluat eine quantitative Duplex-RT-PCR mit den für *C*. *glutamicum* spezifischen Primern CG Forward (SEQ ID NO:1), CG Reverse (SEQ ID NO:2) und der Sonde CG Probe (SEQ ID NO:3) und den für das humane β-Aktin-Gen spezifischen Primern bact100 Forward GAAGGTCTCAAACATGATCTGG (SEQ ID NO:10), bact100 Reverse GGGACGACATGGAGAAAATC (SEQ ID NO:11) und der Sonde bact100 Probe CCCCGTGCTGCTGACCGAG (SEQ ID NO:12) mit den Fluorophoren Hex und BHQ durchgeführt. Die Reaktionsbedingungen wurden gemäß dem Beispiel 3 gewählt, mit der Ausnahme, dass die Primer wurden gemäß der Target DNA wie vorstehend genannt ausgetauscht wurden

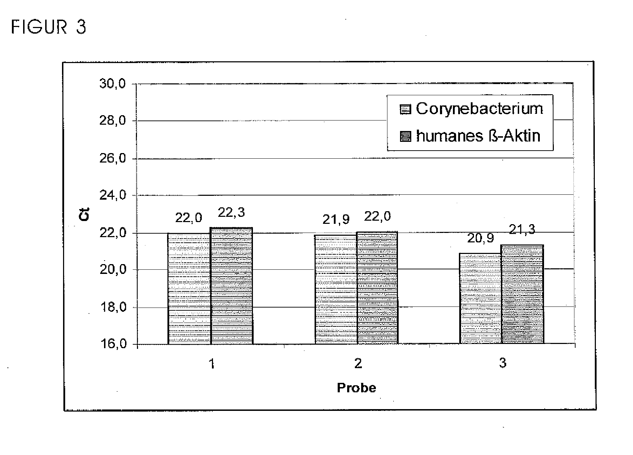

Die Treshold-Cycles (Ct) der quantitativen PCRs der einzeln präparierten Blutproben wurden verglichen. Die Ergebnisse sind in Figur 3 dargestellt. Es zeigt sich, dass die Ct's sowohl für *C. glutamicum* als auch für β-Aktin etwa gleich sind. Dies bedeutet, dass das erfindungsgemäße Präparat die Kontrolle der Reaktionsbedingungen in Präparation und Amplifikation zuverlässig ermöglicht und dabei die Präparation und Amplifikation des Targets Human-β-Aktin-Gen nicht stört.

### Beispiel 5: nachträgliche Behandlung von Tabletten mit unterschiedlichen Zellzahlen von B. subtilis

Tabletten wurden wie bei Tablette C (s. Beispiel 1c) hergestellt mit der Ausnahme, dass 1 ml Phagenüberstand durch 1 ml bidest. Wasser ersetzt wurde, und bei Normalbedingungen über Nacht getrocknet. Anschließend wurden unterschiedliche Mengen *B*. *subtilis* in je 10 µl Kulturmedium auf die getrockneten Tabletten pipettiert. Die Präparate wurden anschließend erneut über Nacht bei Normalbedingungen getrocknet.

Die eingesetzten Zellzahlen waren
a) 5 x 10⁵
b) 1 x 10⁵
c) 5 x 10⁴
d) 1 x 10⁴
e) 5 x 10³
f) 1 x 10³

Die Nukleinsäure-Präparation erfolgte jeweils als Vierfachbestimmung mit je 200 µl Blut und je einer der Tabletten a) bis f) unter Zusatz von 20 µl Proteinase K (Fa. Qiagen). Diese Mischung wurde jeweils 10 Minuten bei 56 °C im Thermomixer geschüttelt. Nach Zugabe von je 200 µl Puffer G (3 M GITC, 20% NP-40) wurde erneut 10 Minuten bei 56 °C im Thermomixer geschüttelt. Die Reinigung erfolgte wie in Beispiel 2 beschrieben durch eine QIAmp Spin Column.

Die quantitative PCR wurde gemäß dem Beispiel 3 mit den für *B. subtilis* spezifischen Primern BS Forward (SEQ ID NO:4), BS Reverse (SEQ ID NO:5) und der Sonde BS Probe (SEQ ID NO:6) sowie mit den für das humane Gen für H 18s spezifischen Primern H18 Forward (SEQ ID NO: 13), H 18 Reverse (SEQ ID NO:14) und der Sonde H18 Probe (SEQ ID NO:15) und den Fluorophoren HEX und BHQ durchgeführt.

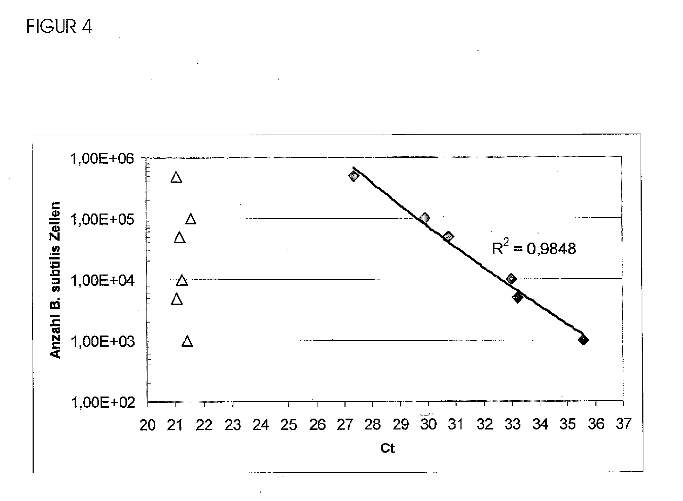

Das Ergebnis ist in Figur 4 dargestellt. Gezeigt sind gemittelten Cts der 4 Versuche mit den Tabletten a) bis f) in Abhängigkeit von den eingesetzten Zellzahlen (gefüllte Raute). Wie anhand der Regressionsgeraden und des angegebenen Bestimmtheitsmaßes zu erkennen ist, besteht ein linearer Zusammenhang zwischen eingesetzter Zellzahl und Ct. Die offenen Dreiecke zeigen die zugehörigen Ct-Werte des humanen H18-Gens. Werte auf gleicher Höhe gehören gemeinsam zur jeweiligen Probe. Die Werte der H18 qPCRs haben alle einen Mittelwert von 21,2 und einen Variationskoeffizienten (CV) von 0,96%.

Es zeigt sich, anhand der Regressionsgeraden in ihrem Bestimmtheitsmaß (R²), sowie des geringen CV, dass die erfindungsgemäßen Präparate auch durch nachträgliche Behandlung der Tabletten mit den Mikroorganismen hergestellt werden können und sehr gut als interner Standard für Nukleinsäurepräparations- und -amplifikationsverfahren geeignet sind. Ein verlässliches Detektionslimit für die erfindungsgemäßen Präparate liegt bei 1000 Zellen pro Tablette.

### Beispiel 6: Reverse Transkription

Unter Zusatz von Tablette C (s. Beispiel 1 c) wurde eine Plasmaprobe wie folgt unter Verwendung des "QIAamp Virus Kits" (Qiagen GmbH, Hilden Deutschland) präpariert und amplifiziert:

1 Tablette C wurde mit 140 µl Plasma und 560 µl Puffer AVL 2 Minuten bei 1400 Upm im Thermomixer gemischt, bis die Tablette gelöst war. Dann wurde 10 Minuten bei Normalbedingungen unter Zusatz von 560 µl Ethanol auf einem Vortex®-Geröt lysiert, Das Gemisch wurde zum Binden 2 mal auf eine QIAamp-Säule gegeben und 1 Minute bei 6000 x g zentrifugiert. Dann wurde zum Waschen mit 500 µl Puffer AW1 1 Minute bei 6000 x g zentrifugiert und anschließend mit 500 µl Puffer AW2 3 Minuten bei maximaler g-Zahl zentrifugiert. Nach 1 Minute Zentrifugieren bei maximaler g-Zahl wurde mit 80 µl Puffer TE eluiert und 1 Minute bei 6000 x g zentrifugiert.

Die anschließend quantitative PCR wurde mit den E.coli Phagen frspezifischen Primern und Sonden fr Forward (SEQ ID NO:7), fr Reverse (SEQ ID NO:8) und fr Probe (SEQ ID NO:9) mit 12,50 µl QIAGEN QuantiTect Probe RT-PCR Mastermix 2x, 0,25 µl QuantiTect RT Mix, 0,10 µl fr Reverse (100 µM), 0,10 µl fr Reverse (100µM), 0,05 µl fr Probe (100 µM), 10,00 µl bidest. Wasser und 2,00 µl Tempfat-Aliquots und 2,00 µl des vorstehend erhaltenen Eluats durchgeführt. Die Temperaturzyklen waren 30 Minuten bei 50 °C, 15 Minuten bei 95 °C und 40 x (15 Sekunden 95 °C, 1 Minute bei 60 °C). Der Versuch wurde insgesamt 24 mal durchgeführt.

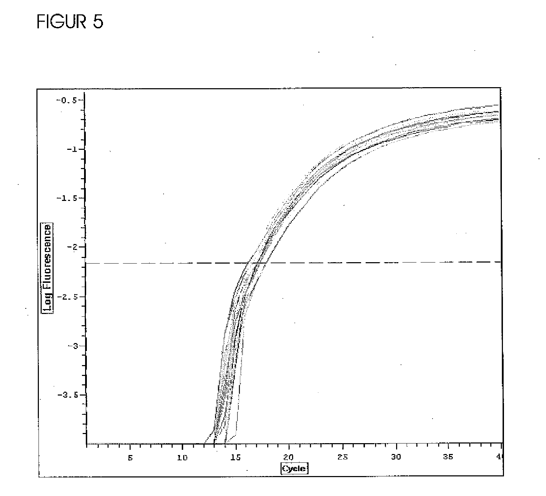

Das Ergebnis ist in Figur 5 dargestellt. Die Figur zeigt die Entwicklung des Fluoreszenzsignals in Abhängigkeit von der Nummer des PCR-Zyklus. 24 einzelne Proben sind gleichzeitig dargestellt. Der Mittelwert der 24 quantitativen PCRs beträgt 17,8 und der Variationskoeffizient CV 2,1 %,

Das Beispiel zeigt, dass die erfindungsgemäßen Präparate hervorragend in reproduzierbarere Weise zur Verwendung als interner Standard in RNA-Präparations- und nachfolgenden RNA-Amplifikationsverfahren geeignet sind.

## Patentansprüche

1. Im wesentlichen festes Produkt, enthaltend mindestens eine Art Biopartikel sowie übliche pharmazeutische Hilfsstoffe.

2. Produkt nach Anspruch 1, wobei die Biopartikel ausgewählt sind aus der Gruppe, die aus Bakterien, Viren, Pilzen, Protozoen, Bakteriophagen, Hefen, Pilzen, Sporen, Parasiten, Pflanzenzellen, tierischen oder menschlichen Zellen, Gameten, Plasmiden und Viroiden besteht.

3. Produkt nach Anspruch 1 oder 2, darüber hinaus enthaltend Puffersubstanzen, Detergenzien, Chelatbildner und/oder Farbstoffe.

4. Produkt nach einem oder mehreren der vorstehenden Ansprüche, wobei die pharmazeutischen Hilfsstoffe ausgewählt sind aus Fließmitteln, Bindemitteln, Sprengmitteln und/oder Füllstoffen.

5. Produkt nach einem oder mehreren der vorstehenden Ansprüche, zusätzlich enthaltend Glaspartikel, Zirkoniumpartikel, magnetische Silikapartikel, Anionaustauscherharz-Partikel und/oder Silziumcarbidpartikel.

6. Produkt nach einem oder mehreren der vorstehenden Ansprüche, zusätzlich enthaltend ein oder mehrere Enzyme.

7. Verfahren zur Herstellung eines im wesentlichen festen Produkts nach einem oder mehreren der vorstehenden Ansprüche, umfassend die folgenden Schritte:
a) Mischen der Bestandteile in den gewünschten Mengen
b) Portionieren auf einem geeigneten Trägermaterial
c) Trocknen.

8. Verfahren zur Herstellung eines im wesentlichen festen Produkts nach einem oder mehreren der Ansprüche 1 bis 6, umfassend die folgenden Schritte:
a) Mischen der Bestandteile in den gewünschten Mengen mit Ausnahme der Biopartikel
b) Portionieren auf einem geeigneten Trägermaterial
c) Trocknen
d) Auftragen der Biopartikel in Form einer Lösung auf den getrockneten Portionen
e) erneutes Trocknen.

9. Verfahren nach Anspruch 7 oder 8, wobei das Trocknen jeweils bei Normalbedingungen durchgeführt wird.

10. Im wesentlichen festes Produkt, erhältlich nach dem Verfahren aus Anspruch 7, 8 oder 9.

11. Verwendung des im wesentlichen festen Produkts nach einem oder mehreren der Ansprüche 1 bis 6 oder 10 als interner Standard bei Nukleinsäurepräparationen mit optional nachfolgenden Nukleinsäurenachweis.

12. Verwendung nach Anspruch 11, wobei die Nukleinsäurepräparationen in einer mikrofluidischen Vorrichtung durchgeführt werden.


SEQUENZPROTOKOLL

<110> QIAGEN GmbH
<120> Produkte, die Biopartikel enthalten, Verfahren zu ihrer Herstellung
und ihre Verwendung als interner Standard in Nukleinsäurepräparations- und
Nukleinsäurenachweis-Verfahren
<130> H 64 071
<160> 15
<170> PatentIn Version 3.1
<210> 1
<211> 23
<212> DNA
<213> Primer
<400> 1
aagctccagc cacccaaaac tac 23

<210> 2
<211> 23
<212> DNA
<213> Primer
<400> 2
ctaccaacca ctaatgcgtc gtc 23

<210> 3
<211> 22
<212> DNA
<213> Sonde
<400> 3
atcgccttcc agacgctcaa cg 22

<210> 4
<211> 24
<212> DNA
<213> Primer

<400> 4
acatcttacc gcaactacga ccat                                        24

<210> 5
<211> 24
<212> DNA
<213> Primer
<400> 5
tagcatagtc tttgtcccac cgta                                        24

<210> 6
<211> 23
<212> DNA
<213> Sonde
<400> 6
ggaggcgatc tatgtcttgt cca                                         23

<210> 7
<211> 23
<212> DNA
<213> Primer
<400> 7
cttctgatcc gcatagtgac gac                                         23

<210> 8
<211> 22
<212> DNA
<213> Primer
<400> 8
aacggtcatt cgcctccagc ag                                          22

<210> 9
<211> 23
<212> DNA

<213>  Sonde
<400>  9
taggggatgg taacgacgaa gca                                    23

<210>  10
<211>  22
<212>  DNA
<213>  Primer
<400>  10
gaaggtctca aacatgatct gg                                     22

<210>  11
<211>  20
<212>  DNA
<213>  Primer
<400>  11
gggacgacat ggagaaaatc                                        20

<210>  12
<211>  19
<212>  DNA
<213>  Sonde
<400>  12
ccccgtgctg ctgaccgag                                         19

<210>  13
<211>  22
<212>  DNA
<213>  Primer
<400>  13
gccgctagag gtgaaattct tg                                     22

<210>  14
<211>  21

<212> DNA
<213> Primer
<400> 14
cattcttggc aaatgctttc g 21

<210> 15
<211> 21
<212> DNA
<213> Sonde
<400> 15
accggcgcaa gacggaccag a 21